# EUROPÄISCHE PATENTSCHRIFT

(11) **EP 3 844 397 B1**
(45) Veröffentlichungstag und Bekanntmachung des Hinweises auf die Patenterteilung: **12.10.2022**
(21) Anmeldenummer: 19762922.3
(22) Anmeldetag: 29.08.2019
(51) Int. Cl.: F04D 9/00, F04D 15/00, F04D 29/66, A61M 60/50

(54) **BETRIEB EINER KREISELPUMPE ZUR ENTFERNUNG VON GASANSAMMLUNGEN**
OPERATION OF A CENTRIFUGAL PUMP FOR THE REMOVAL OF GAS BUILDUP
RÉGIME DE FONCTIONNEMENT D'UNE POMPE CENTRIFUGE POUR L'ÉLIMINATION D'ACCUMULATIONS DE GAZ

(30) Priorität: 30.08.2018 DE 102018006877
(43) Veröffentlichungstag der Anmeldung: 07.07.2021
(73) Patentinhaber: Fresenius Medical Care Deutschland GmbH, 61352 Bad Homburg (DE)
(72) Erfinder: HEIDE, Alexander, 65817 Eppstein (DE)
(74) Vertreter: Dreyhsig, Jörg
(86) Internationale Anmeldenummer: PCT/EP2019/073055
(87) Internationale Veröffentlichungsnummer: WO 2020/043811

(56) Entgegenhaltungen:
- US-A1- 2016 084 254
- US-A1- 2016 222 969

## Beschreibung

Die vorliegende Erfindung liegt auf dem Gebiet der Pumpvorrichtung zum Pumpen von Flüssigkeiten aufweisend eine Zentrifugalpumpe mit radial pumpendem Pumpenrad mit hohlem Zentrum.

Bekannte Zentrifugalpumpen und Pumpvorrichtungen zum Pumpen mit Zentrifugalpumpen weisen rotationsfähige Bestandteile im Inneren eine Pumpenkopfes auf, durch deren Rotation ein zu förderndes Medium mitgerissen und somit auch in Rotation versetzt wird. Durch die Rotationsbewegung wirken Zentripetalkräfte, welche sich in einem rotierenden Bezugssystem als Zentrifugalkräfte äußern. Wenn das zu pumpende Medium einigermaßen mittig senkrecht zur Rotationsebene in das Innere einer Pumpe gelangt, wird es durch die Zentrifugalkräfte infolge der Rotation nach außen gedrückt und dadurch gefördert.

Bekannte Zentrifugalpumpen mit radial pumpendem Pumpenrad werden unter anderem zur Förderung von gasförmigen Medien, z.B. Luft zum Kühlen oder zur Frischluftzufuhr, und zur Förderung von flüssigen Medien, z.B. als Treibstoffförderpumpe, zur Förderung von Prozesschemikalien und für medizinische Anwendungen, eingesetzt.

Zentrifugalpumpen werden auch Kreiselpumpen genannt. Radialpumpen sind Zentrifugalpumpen, bei denen das zu fördernde Medium radial aus dem Laufrad austritt. Das Laufrad einer Pumpe wird bisweilen auch als Pumpenrad bezeichnet.

Die vorliegende Anmeldung befasst sich mit Pumpvorrichtung zum Pumpen von Flüssigkeiten aufweisend eine Zentrifugalpumpe mit radial pumpendem Pumpenrad mit hohlem Zentrum sowie mit Zentrifugalpumpe mit radial pumpendem Pumpenrad mit hohlem Zentrum, welche bei Raumtemperatur Flüssigkeiten betrieben werden können und welche bei Raumtemperatur Flüssigkeiten fördern können, insbesondere zum Einsatz in Medizingeräten. Solche Pumpen können beispielsweise aktiv elektronisch geregelt levitierende Impellerpumpen sein.

Zentrifugalpumpen mit radial pumpendem Pumpenrad mit hohlem Zentrum sind Radialpumpen, welche der übergeordneten Kategorie von Kreiselpumpen zugeordnet werden.

Typischerweise ist die Massendichte von Flüssigkeiten, die von flüssigkeitsfördernden Zentrifugalpumpen mit radial pumpendem Pumpenrad mit hohlem Zentrum und Pumpvorrichtungen mit Zentrifugalpumpe mit radial pumpendem Pumpenrad mit hohlem Zentrum gefördert werden, höher, als die Massendichte von Gasbeimischungen in den damit geförderten Flüssigkeiten. Solche Gasbeimischungen können originär in den Flüssigkeiten vorliegen oder z.B. durch relative Unterdrücke auf der Ansaugseite von Pumpvorrichtungen aus dem die Vorrichtung umgebenden Medium - wie z.B. Luft oder ein Prozessgas - angesaugt werden. Infolge der geringeren Massendichte der Gasbeimischungen und des zentrifugalen Wirkprinzips der Pumpen kann es daher beim Betrieb solcher Pumpen und Pumpvorrichtungen zur Ansammlung von Gas im Inneren des rotierenden Bereichs der Zentrifugalpumpen kommen. Die Gasbeimischungen in den Flüssigkeiten können bereits unerwünscht sein. Die Gasansammlung im Inneren der Pumpe ist aber sicher unerwünscht, da dadurch beispielsweise die Pumpleistung der Pumpen eingeschränkt werden kann. Im schlimmsten Fall kann die Pumpleistung wegen einer Gasansammlung im Inneren einer Pumpe soweit abfallen, dass auf der Ansaugseite nicht mehr ausreichend Unterdruck erzeugt wird, um die zu pumpende Flüssigkeit noch weiter zu pumpen. Zum Stand der Technik, der diese Problematik bespricht, gehören die Dokumente US 2016/084254 A1 und US 2016/222969 A1.

Es ist daher eine Aufgabe der vorliegenden Erfindung, eine Vorrichtung zum Pumpen von Flüssigkeiten mit einer Zentrifugalpumpe mit radial pumpendem Pumpenrad mit hohlem Zentrum bereitzustellen, welche dazu eingerichtet ist, Gasansammlungen aus dem Inneren der Pumpe zu entfernen.

Diese Aufgabe wird durch die Pumpvorrichtung zum Pumpen von Flüssigkeiten aufweisend eine Zentrifugalpumpe mit radial pumpendem Pumpenrad mit hohlem Zentrum gemäß Anspruch 1 und vom Verfahren zur Entfernung von Gasansammlungen aus dem Inneren von Zentrifugalpumpen mit radial pumpendem Pumpenrad mit hohlem Zentrum gemäß Anspruch 12 gelöst. Ausgestaltungen und Weiterbildungen des Erfindungsgedankens sind Gegenstand von abhängigen Ansprüchen.

Es werden eine Pumpvorrichtung zum Pumpen von Flüssigkeiten aufweisend eine Zentrifugalpumpe mit radial pumpendem Pumpenrad mit hohlem Zentrum, eine Pumpvorrichtung zur Beförderung von Blut und / oder medizinischer Behandlungsflüssigkeit und / oder medizinischer Behandlungs-Abfallflüssigkeit, ein Medizinisches Behandlungsgerät, wobei eine Pumpvorrichtung mit Zentrifugalpumpe mit radial pumpendem Pumpenrad mit hohlem Zentrum zur Förderung von Blut oder einer medizinischen Behandlungsflüssigkeit oder einer medizinischer Behandlungs-Abfallflüssigkeit eingesetzt wird, und ein Verfahren zur Entfernung von Gasansammlungen aus dem Inneren von Zentrifugalpumpen mit radial pumpendem Pumpenrad mit hohlem Zentrum offenbart.

Die Pumpvorrichtung zum Pumpen von Flüssigkeiten weist zumindest eine Zentrifugalpumpe mit radial pumpendem Pumpenrad mit hohlem Zentrum, eine Erfassungsvorrichtung zur Erfassung eines ersten Betriebsparameters, einen Sensor zur Messung eines zweiten Betriebsparameters und ein Steuergerät zur Steuerung der Zentrifugalpumpe auf. Der Pumpe der Pumpvorrichtung kann im Betrieb zu jedem Zeitpunkt ein Wert des ersten Betriebsparameters zugeordnet werden. Das Steuergerät der Pumpvorrichtung ist zumindest mit der Pumpe, der Erfassungsvorrichtung zur Erfassung des ersten Betriebsparameters und mit dem Sensor verbunden. Ferner ist das Steuergerät dazu eingerichtet, dass es den Betrieb der Pumpe steuert. Außerdem ist das Steuergerät dazu eingerichtet, dass es im Flüssigkeitsförderbetrieb der Pumpe Gasansammlungen aus dem hohlen Zentrum der Pumpe entfernen kann. Das Steuergerät kann Gasansammlungen entfernen, indem es in einem ersten Schritt zur Gasentfernung die Pumpe so betreibt, dass zumindest der erste Betriebsparameter der Pumpe über die Zeit variiert. Das variieren über die Zeit bedeutet, dass der erste Betriebsparameter nacheinander eine Reihe von verschiedenen vorbestimmten Werten einnimmt. Während die Pumpe so betrieben wird, dass der erste Betriebsparameter variiert, werden zu den Werten des ersten Betriebsparameters jeweils die zugeordneten Werte des zweiten Betriebsparameters aufgezeichnet, d.h. Wertepaare werden abgelegt. Das sind immer Paare eines Wertes des ersten Betriebsparameters und des zu dem ersten Wert zugeordneten zweiten Betriebsparameters. In einem zweiten Schritt zur Gasentfernung werden lokale und / oder globale Extrema der zuvor aufgezeichneten Werte des zweiten Betriebsparameters bestimmt. In einem dritten Schritt zur Gasentfernung werden diejenigen Werte des ersten Betriebsparameters bestimmt, welche den zuvor ermittelt Extrema des zweiten Betriebsparameters zugeordnet sind. In einem vierten Schritt zur Gasentfernung wird die Pumpe so betrieben, dass sie für eine vorbestimmte Zeitdauer zumindest einen derjenigen zuvor bestimmten Werte des ersten Betriebsparameters annimmt, welche den Extrema des zweiten Betriebsparameters zugeordnet sind.

Das Verfahren zur Entfernung von Gasansammlungen aus dem Inneren von Zentrifugalpumpen mit radial pumpendem Pumpenrad mit hohlem Zentrum zeichnet sich dadurch aus, dass es mindestens die folgenden Schritte aufweist, nämlich:
- Betreiben einer Zentrifugalpumpe, wobei ein erster Betriebsparameter der Pumpe dahingehend variiert wird, dass der erste Betriebsparameter nacheinander eine Reihe von verschiedenen vorbestimmten Werten einnimmt, Währenddessen wird zu jedem Wert des ersten Betriebsparameters zumindest ein Wert eines zweiten Betriebsparameters aufgezeichnet.
- Bestimmung von lokalen und / oder globalen Extrema der zuvor aufgezeichneten Werte des zweiten Betriebsparameters,
- Bestimmung derjenigen Werte des ersten Betriebsparameters, welche den zuvor ermittelt Extrema des zweiten Betriebsparameter zugeordnet sind,
- Betreiben der Pumpe so, dass sie für eine vorbestimmte Zeitdauer einen der den Extrema des zweiten Betriebsparameters zugeordneten Werte des ersten Betriebsparameters annimmt.

Der Erfindung liegt die Erkenntnis zugrunde, dass sich Extrema des zweiten Betriebsparameters ergeben, wenn der erste Betriebsparameter so eingestellt ist, dass besonders günstige Bedingungen für den Transport bzw. das Fördern von unerwünschten Gasansammlungen vorliegen. Solche besonders günstige Bedingungen können beispielsweise Resonanzbedingungen im Inneren der Pumpe sein. Ein Paar aus einem Extremwert des zweiten Betriebsparameters und dem zugeordneten Wert des ersten Betriebsparameters kann also eine Resonanzbedingung ausmachen, die besonders gut für das Pumpen von Gas und damit für das Entfernen von unerwünschten Gasansammlungen aus der Pumpe geeignet ist. Um unerwünschte Gasansammlungen aus dem Inneren der Pumpe zu entfernen, ist es notwendig, die Pumpe mit Betriebsparametern zu betreiben, die besonders dazu geeignet sind, Gas aus dem Inneren der Pumpe heraus zu pumpen, zu transportieren, zu fördern. Es handelt sich ja um eine Pumpvorrichtung zum Pumpen von Flüssigkeiten. Eine solche Vorrichtung ist also in erster Linie dazu eingerichtet, Flüssigkeiten zu pumpen. Um damit Gas zu pumpen / transportieren / fördern, z.B. um unerwünschte Gasansammlungen aus der Pumpvorrichtung selbst zu entfernen, sind besondere Maßnahmen zu ergreifen, nämlich bestimmte für diesen Zweck optimierte Parameter zu finden und anzulegen. Durch den normalen Betrieb der Vorrichtung kommt es zur unerwünschten Ansammlung von Gas im Pumpeninneren, gerade weil im gewöhnlichen Betrieb der Vorrichtung die Gasansammlungen nicht aus dem Inneren der Pumpe herausgepumpt werden.

Eine Zentrifugalpumpe mit radial pumpendem Pumpenrad mit hohlem Zentrum kann eine Impellerpumpe sein. Dabei ist ein Impeller das Pumpenrad. Das Pumpenrad wird auch Laufrad der Pumpe genannt. Beispielsweise kann ein solches Pumpenrad im Wesentlichen scheibenförmig sein und im Wesentlichen radial verlaufende Schaufelblätter aufweisen, welche ein zu förderndes Pumpmedium bei einer Drehbewegung des Pumpenrads mitnehmen. Das von der Scheibenebene entfernte Ende der Schaufelblätter kann wiederum mit einer weiteren, parallel zur ersten Scheibe angeordneten Scheibe verbunden sein. Eine Ausführung mit zwei parallel zueinander angeordneten Scheiben ist ebenso denkbar wie eine Ausführung mit nur einer Scheibe, an der die Schaufelblätter angeordnet sind, währenddessen das scheibenferne Ende der Schaufelblätter frei ist. Eine erste Variante einer solchen Scheibe weist eine zentrale Ausnehmung auf, ist also grob gesagt ringförmig. Eine alternative Variante einer solchen Scheibe ist als Kreisscheibe ausgeführt, weist also gerade keine zentrale Ausnehmung auf.

Im Kontext der vorliegenden Anmeldung ist unter einer Zentrifugalpumpe mit hohlem Zentrum zu verstehen, dass die Schaufelblätter sich zum Zentrum hin nicht bis zur Rotationsachse erstrecken, sondern dass ein makroskopisch erkennbarer Bereich an und / oder unmittelbar um die Rotationsachse des Pumpenrads herum frei von Schaufelblättern ist. Das hohle Zentrum ist aber auch nicht mit einem festen Volumenkörper, etwa einem massiven Zylinder, ausgefüllt. Vielmehr ist das Zentrum hohl, sodass die zu pumpende Flüssigkeit durch das hohle Zentrum strömen kann. Es ist denkbar, dass ein massiver Körper genau auf der Rotationsachse des Pumpenrads liegt, z.B. eine Achse oder eine Welle. Dann ist das hohle Zentrum der zentral um die Rotationsachse liegende Bereich zwischen dem massiven Körper und den Schaufelblättern - also der Bereich, den die zu pumpende Flüssigkeit frei durchströmen kann.

Im Kontext der Erfindung kann die Vorrichtung die Gasentfernung zum Zeitpunkt des initialen Befüllens eines Flüssigkeitssystems ("Priming") oder während des Betriebs, insbesondere auch während eines regulären Pumpbetriebs z.B. eines medizinischen Behandlungsgerätes stattfinden.

Ein Erster Betriebsparameter einer vorliegenden Vorrichtung kann ein Pumpenparameter, d.h. ein dem Betrieb der Pumpe der Vorrichtung zuzuordnender Parameter sein.

In allen Ausführungsformen der vorliegenden Erfindung kann eine Vorrichtung zur Erfassung des ersten Betriebsparameters ein Teil des Antriebs, beispielsweise Teil der Leistungselektrik oder der Leistungselektronik, einer Zentrifugalpumpe sein. Beispielsweise kann der Antriebsstrom der Pumpe der erste Betriebsparameter sein. Ein Sensor zur Erfassung eines Magnetfelds kann eine Vorrichtung zur Erfassung des ersten Betriebsparameters sein. Das kann beispielsweise ein Hallsensor sein.

Ein Zweiter Betriebsparameter der Vorrichtung kann ein Zustandsparameter der Vorrichtung sein. Eine vorgeschlagene Vorrichtung verfügt über einen Sensor zur Messung eines zweiten Betriebsparameters.

Gasansammlungen im Inneren der Pumpe sind unerwünscht. Erwünscht ist, dass das gesamte Innenvolumen der Pumpe nur mit der zu pumpenden Flüssigkeit gefüllt ist.

Eine Vorrichtung, wie Sie in der vorliegenden Anmeldung vorgeschlagen wird, ist dazu eingerichtet, Gasansammlungen aus dem hohlen Zentrum der Pumpe zu entfernen. Das heißt aber nicht, dass im typischen Betrieb einer solchen Vorrichtung ununterbrochen Gasansammlungen aus dem Inneren der Pumpe entfernt werden. Vielmehr wird das Entfernen von Gasansammlungen bedarfsgerecht erfolgen.

Typische Werte für vorbestimmte Zeitdauern T, die eine Pumpe zur Entfernung unerwünschter Gasansammlungen mit einem bestimmten Wert des ersten Betriebsparameters betrieben wird liegen beispielsweise im Bereich von 0,01 bis 1000 Sekunden. Bevorzugt liegen die Zeitdauern im Bereich von 1 bis 10 Sekunden, beispielsweise 3 Sekunden oder 5 Sekunden. In anderen Worten können vorbestimmte Zeitdauern T, die eine Pumpe zur Entfernung unerwünschter Gasansammlungen so betrieben wird, dass sie den Extrema des zweiten Betriebsparameters zugeordnete Werte des ersten Betriebsparameters einnimmt, beispielsweise im Bereich von 0,01 bis 1000 Sekunden, bevorzugt im Bereich von 1 bis 10 Sekunden, beispielsweise 3 Sekunden oder 5 Sekunden, liegen.

Ein Sensor zur Messung eines zweiten Betriebsparameters kann auch an oder in einer an die Pumpvorrichtung oder an die Pumpe angeschlossenen Flüssigkeitsleitung angeordnet sein. Dabei kommt eine Anordnung, die als im Betrieb stromaufwärts angeordnet bezeichnet werden kann, genauso in Frage wie eine Anordnung, die als im Betrieb stromabwärts angeordnet bezeichnet werden kann, in Frage. Es ist auch denkbar, dass bevorzugte Ausführungsformen der hier vorgeschlagenen Pumpvorrichtung mehr als einen Sensor zur Messung eines zweiten Betriebsparameters aufweisen, z.B. einen Sensor in unmittelbarer Nähe der Pumpe und einen zweiten Sensor, der von der Pumpe entfernt an einer daran angeschlossenen Flüssigkeitsleitung angeordnet ist. Oder eine hier vorgeschlagene Pumpvorrichtung kann über mehrere Sensoren verfügen, sodass sowohl stromaufwärts als auch stromabwärts der Pumpe ein zweiter Betriebsparameter gemessen werden kann. Ein Sensor zur Messung eines zweiten Betriebsparameters kann beispielsweise ein Körperschallsensor sein.

Die Extrema des zweiten Betriebsparameters markieren erfindungsgemäß eine Betriebseinstellung, bei der besonders effektiv Gasansammlungen durch die Pumpenvorrichtung transportiert werden können. Wenn im Pumpbetrieb in der Pumpe eine Resonanz in den im Förderraum der Pumpe befindlichen Medien eintritt, kann bei einem Betrieb in Resonanzbedingung der zweite Betriebsparameter einen Extremwert annehmen. In anderen Worten können die durch Extrema im zweiten Betriebsparameter ausgezeichneten Wertepaare von erstem Betriebsparameter und zweitem Betriebsparameter beispielsweise mit einer Resonanz in den Medien im Förderraum der Pumpe, also auch im hohlen Zentrum des Pumpenrads zusammenhängen.

Eine besonders vorteilhafte Ausführungsform einer hier vorgeschlagenen Pumpvorrichtung ist so eingerichtet, dass der erste Betriebsparameter eine Pumpendrehzahl pro Zeiteinheit oder eine Pumpenfrequenz der Zentrifugalpumpe ist. Dadurch ergibt sich der besondere Vorteil, dass eine Einstellung einer direkten Betriebskenngröße aufgefunden wird, bei der unerwünschte Gasansammlungen besonders gut transportiert werden können.

Bei einer weiteren besonders vorteilhaften Ausführungsform einer hier vorgeschlagenen Pumpvorrichtung ist der zweite Betriebsparameter direkt oder indirekt an der Pumpvorrichtung gemessener Körperschall oder Schall in der zu pumpenden Flüssigkeit. Durch die Messung von Körperschall oder Schall in der Flüssigkeit lassen sich besonders vorteilhaft einfach oder eindeutig Betriebsbedingungen für einen optimalen Transport von unerwünschten Gasansammlungen identifizieren.

Bei einer weiteren besonders vorteilhaften Ausführungsform einer hier vorgeschlagenen Pumpvorrichtung ist die Pumpe elektrisch angetrieben und ein erster Betriebsparameter ist ein elektrischer Betriebsstrom der Pumpe oder ein Betriebsstrom pro Drehzahl der Pumpe. Dadurch ergibt sich der besondere Vorteil, dass einerseits ein solcher Betriebsparameter ohne größeren technischen Aufwand aus üblichen elektrischen Pumpenantrieben leicht gewonnen werden kann und dass andererseits ein direkt mit dem Pumpenbetrieb korrelierender Parameter herangezogen wird.

Bei einer weiteren besonders vorteilhaften Ausführungsform einer hier vorgeschlagenen Pumpvorrichtung ist die Pumpe eine magnetisch levitierend gelagerte Impellerpumpe und ein erster Betriebsparameter ein Lagerstrom der Pumpe oder ein Lagerstrom pro Drehzahl der Pumpe ist. Magnetisch levitierend gelagert Impellerpumpen sind für bestimmte Anwendungen besonders geeignet. Insbesondere ist eine levitierende Lagerung praktisch verschleißfrei und kontaktlos. Durch das Heranziehen von Lagerstrom der Pumpe oder Lagerstrom pro Drehzahl der Pumpe als ersten Betriebsparameter ergibt sich der besondere Vorteil, dass einerseits ein solcher Betriebsparameter ohne größeren technischen Aufwand aus Pumpenantrieben magnetisch levitierender Pumpen leicht gewonnen werden kann und dass andererseits ein direkt mit dem Pumpenbetrieb korrelierender Parameter herangezogen wird.

Bei einer weiteren besonders vorteilhaften Ausführungsform einer hier vorgeschlagenen Pumpvorrichtung ist das Steuergerät dazu eingerichtet, nach der Durchführung des vierten Schrittes erneut unerwünschte Gasansammlungen zu entfernen, indem die Schritte zur Entfernung von Gasansammlung vom ersten Schritt bis zum vierten Schritt erneut durchlaufen werden, wobei die ausgezeichneten Werte des ersten Betriebsparameters verschiedener Durchläufe unterschiedlich sein können. Da sich Extrema des zweiten Betriebsparameters ergeben, wenn der erste Betriebsparameter so eingestellt ist, dass besonders günstige Bedingungen für den Transport bzw. das Fördern von unerwünschten Gasansammlungen vorliegen, ermöglicht diese Ausführungsform besonders vorteilhaft eine vollständige Entfernung von unerwünschten Gasansammlungen: Bei einer teilweisen Entfernung unerwünschter Gasansammlungen kann sich eine Veränderung der Resonanzbedingungen im Inneren der Pumpe ergeben. Infolgedessen kann sich verändern, bei welchen Werten des ersten Betriebsparameters Extrema des zweiten Betriebsparameters vorliegen. Nachdem also mit einer ersten Resonanzbedingung mit einem ersten Paar von erstem Betriebsparameter und zweitem Betriebsparameter unerwünschte Gasansammlungen aus der Pumpe gefördert wurden, können sich die Resonanzbedingungen verschieben. Eine erneute Ermittlung der optimalen Bedingungen zur Entfernung von unerwünschten Gasansammlungen kann besonders vorteilhaft eine vollständige, eine weitestgehend vollständige oder eine besonders effiziente Entfernung von unerwünschten Gasansammlungen ermöglichen. Es kann auch besonders vorteilhaft sein, mehr als zweimal neue Extrema des zweiten Betriebsparameters in Abhängigkeit vom ersten Betriebsparameter zu bestimmen. Wenn eine Veränderung der Werte des zweiten Parameters, bei denen Maxima vorliegen, über verschiedene Durchläufe erkannt wird, kann diese Veränderung beispielsweise in einer Protokolldatei abgelegt werden. Wenn Maxima unverändert bestehen bleiben, kann das als Misserfolg in Hinsicht auf das Entfernen unerwünschter Gasansammlungen gedeutet und in einer Protokolldatei abgelegt werden.

Bei einer weiteren besonders vorteilhaften Ausführungsform einer hier vorgeschlagenen Pumpvorrichtung ist das Steuergerät dazu eingerichtet, den vierten Schritt für mehrere unterschiedliche den Extrema des zweiten Betriebsparameters zugeordneten Werte des ersten Betriebsparameters durchzuführen. Es kann besonders vorteilhaft zeitersparend sein, wenn eine erfindungsgemäße Vorrichtung direkt nacheinander an mehreren Werten des ersten Betriebsparameters betrieben wird, welche Extrema des zweiten Betriebsparameters zugeordnet sind, ohne zwischendurch erneut Extrema zu ermitteln. Dadurch kann besonders vorteilhaft beispielsweise der Zeitbedarf für die erneute Ermittlung der Extrema eingespart werden und zugleich kann besonders effizient Gas aus dem Inneren der Pumpe entfernt werden, weil mehrere ermittelte Werte des ersten Betriebsparameters eingenommen werden.

Bei einer weiteren besonders vorteilhaften Ausführungsform einer hier vorgeschlagenen Pumpvorrichtung ist das Steuergerät dazu eingerichtet, den ersten Betriebsparameter im ersten Schritt linear oder in Sprüngen mit festen Abständen oder als Intervallschachtelung, welche sich einem vorbestimmten Wert in vorbestimmter Geschwindigkeit näher, zu variieren. Eine lineare Variation bringt besonders vorteilhaft einen einfachen Zusammenhang zwischen dem ersten Betriebsparameter und dem zweiten Betriebsparameter mit sich. Eine Variation in festen Sprüngen bringt eine besonders zeitsparende Ermittlung von Extrema als Vorteil mit sich. Eine Variation mit einer Intervallschachtelung zur Annäherung kann besonders vorteilhaft zeitsparend sein und /oder besonders genaue Kenntnis der Extrema ermöglichen. Bei einer besonders vorteilhaften Weiterbildung ist die Vorrichtung dazu eingerichtet, nach einer Variation des ersten Betriebsparameters in eine Richtung (also z.B. ein Rampenscan von einem kleinen zu einem größeren Wert hin) zusätzlich oder alternativ eine Variation in entgegengesetzter Richtung durchzuführen. Dadurch können vorteilhaft Messfehler verringert und die Genauigkeit verbessert werden. In vorteilhaften Weiterbildungen der Vorrichtungen ist das Steuergerät dazu eingerichtet, nach einem gröberen Durchlaufen der Variation (grober Scan) eine Variation mit kleineren Schritten durchzuführen (feiner Scan). In vorteilhaften Weiterbildungen ist das Steuergerät dazu eingerichtet, die Variation des ersten Betriebsparameters in Abhängigkeit von weiteren Parametern der geförderten Flüssigkeit, z.B. von deren Temperatur, Viskosität etc. abhängig einzustellen.

Bei einer weiteren besonders vorteilhaften Ausführungsform einer hier vorgeschlagenen Pumpvorrichtung ist das Steuergerät dazu eingerichtet, die Gegenwart einer unerwünschten Gasansammlung im Inneren der Pumpe zu erkennen, indem beim Betrieb der Pumpe kontinuierlich oder in vorbestimmten Zeitintervallen der erste Betriebsparameter der Pumpe überwacht und dessen Veränderung über die Zeit mit vorbestimmten Erkennungsprofilen verglichen wird, und bei Erkennen einer Gasansammlung das Entfernen von Gasansammlungen durchzuführen. Dadurch kann besonders vorteilhaft ermöglicht werden, eine Benachrichtigung oder einen Alarm bezüglich einer entdeckten Gasansammlung auszugeben. Weiterhin kann dadurch besonders vorteilhaft in einer Weiterbildung der Vorrichtung ermöglicht werden, eine Vorrichtung bereitzustellen, wobei das Steuergerät ferner dazu eingerichtet ist, Gasansammlungen im Inneren der Pumpe zu erkennen und automatisch oder nach einer Bestätigung durch einen Benutzer unerwünschte Gasansammlungen aus dem Inneren der Pumpe zu entfernen.

Bei einer weiteren besonders vorteilhaften Ausführungsform einer hier vorgeschlagenen Pumpvorrichtung ist das Steuergerät dazu eingerichtet, den normalen

Flüssigkeitsförderbetrieb fortzusetzen, wenn beim Entfernen von Gasansammlungen im zweiten Schritt keine Extrema aufgefunden worden sind. Dadurch wird besonders vorteilhaft ermöglicht, in Abwesenheit einer Gasansammlung ohne weiteren Zeitverlust den normalen Betrieb fortzusetzen. Diese Ausführungsform eignet sich besonders für einen Betrieb, bei dem routinemäßig nach Extrema des zweiten Betriebsparameters gesucht wird.

Eine besonders vorteilhaften Ausführungsform einer hier vorgeschlagenen Pumpvorrichtung ist zur Beförderung von Blut und / oder medizinischer Behandlungsflüssigkeit und / oder medizinischer Behandlungs-Abfallflüssigkeit eingerichtet. Die vorgeschlagene Vorrichtung ist mit besonderem Vorteil zum Einsatz zur Beförderung von Blut, z.B. bei extrakorporalen Blutbehandlungstherapien wie Hämodialyse, Hämofiltration, Hämodiafiltration und Apherese aber auch bei Herzunterstützungstherapien geeignet. Das betrifft sowohl Behandlungssystem zur chronischen Therapie als auch Systeme zur akuten Behandlung auf Intensivstationen. Eine hier vorgeschlagene Vorrichtung ist auch besonders vorteilhaft zum Pumpen von frischem Dialysat, von verbrauchtem Dialysat, von frischer Aphereseflüssigkeit und von verbrauchter Aphereseflüssigkeit geeignet.

### Kurze Beschreibung der Zeichnung

Im Folgenden werden die Vorrichtung und das Verfahren mit Bezug auf die Zeichnung beschrieben. In der Zeichnung zeigt:
- Figur 1: die erfindungsgemäße Pumpvorrichtung zum Pumpen von Flüssigkeiten aufweisend eine Zentrifugalpumpe mit radial pumpendem Pumpenrad mit hohlem Zentrum schematisch in einer ersten Ausführungsform,
- Figur 2: eine erfindungsgemäße Pumpvorrichtung, bei der gegenüber Figur 1 die Erfassungsvorrichtung zur Erfassung des ersten Betriebsparameters Bestandteil des Steuergeräts ist oder im Steuergerät angeordnet ist,
- Figur 3: eine erfindungsgemäße Pumpvorrichtung, bei der gegenüber Figur 1 der Sensor zur Messung eines zweiten Betriebsparameters von der Pumpe beabstandet an oder in einer Flüssigkeitsleitung stromabwärts der Pumpe angeordnet ist, die mit der Pumpvorrichtung und der Pumpe verbunden ist,
- Figur 4: eine erfindungsgemäße Pumpvorrichtung, bei der gegenüber Figur 1 der Sensor zur Messung eines zweiten Betriebsparameters von der Pumpe beabstandet an oder in einer Flüssigkeitsleitung stromaufwärts der Pumpe angeordnet ist, die mit der Pumpvorrichtung und der Pumpe verbunden ist,
- Figur 5: ein beispielhaftes Pumpenrad mit hohlem Zentrum zum radialen Pumpen,
- Figur 6: die erfindungsgemäße Entfernung von Gasansammlungen schematisch in vier Schritten,
- Figur 7: eine Variante der erfindungsgemäßen Entfernung von Gasansammlungen schematisch, wobei auch eine Erkennung von Gasansammlungen im regulären Betrieb und eine Überprüfung des Erfolgs der Entfernung vorgesehen sind,
- Figur 8: eine erfindungsgemäße Variation des ersten Betriebsparameters und die simultane Aufzeichnung des zweiten Betriebsparameters sowie ein Extremum im zweiten Parameter. Figur 8a zeigt ein negatives Extremum und Figur 8b ein positives Extremum.

In den Figuren können gleiche oder ähnliche Elemente mit denselben Bezugszeichen referenziert sein.

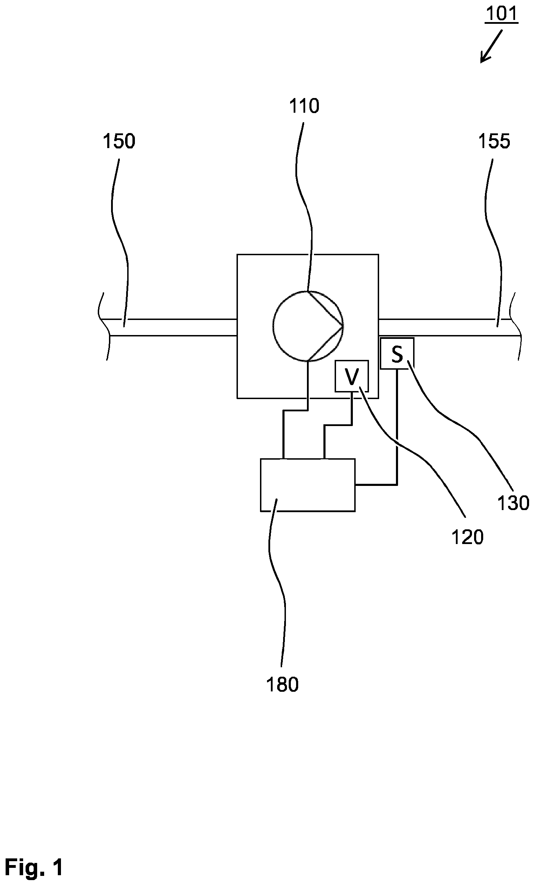

Figur 1 zeigt eine erste Ausführung einer erfindungsgemäßen Pumpvorrichtung 101 schematisch. Die Pumpvorrichtung 101 zum Pumpen von Flüssigkeiten weist eine Zentrifugalpumpe 110 mit radial pumpendem Pumpenrad mit hohlem Zentrum auf. Zumindest ein erster Betriebsparameter 510 kann der Pumpe 110 im Betrieb zu jeder Zeit zugeordnet werden. Als eine Möglichkeit ist in der Figur angedeutet, dass Flüssigkeitsleitungen 150, 155 an die Pumpvorrichtung 101 angeschlossen sind: Eine erste Leitung 150 führt das Pumpmedium, d.h. die zu pumpende Flüssigkeit, zur Zentrifugalpumpe 110 hin und eine zweite Leitung 155 führt die zu pumpende Flüssigkeit von der Zentrifugalpumpe 110 ab. Die erste Leitung 150 ist von der Pumpe 110 aus gesehen also stromaufwärts, während die zweite Leitung 155 von der Pumpe 110 aus gesehen stromabwärts verläuft. Es sind andere Möglichkeiten denkbar, eine hier vorgeschlagene Pumpvorrichtung mit Flüssigkeitsleitungen anzuschließen. Die gezeigten Anschlüsse sind eine Variante und nicht limitierend zu sehen. Die Pumpvorrichtung weist weiterhin eine Erfassungsvorrichtung 120 zur Erfassung des ersten Betriebsparameters 510 auf. In der gezeigten Ausführungsform ist die Erfassungsvorrichtung 120 an der Pumpe 110 angebracht oder in deren Nähe angeordnet. Ein Beispiel für eine solche Vorrichtung weist beispielsweise einen Hall-Sensor auf, der im Fall einer Pumpe 110, durch deren Betrieb bedingt zeitlich veränderliche Magnetfelder auftreten. Ein an oder in der Nähe der Pumpe 110 angeordneter Hall-Sensor kann dazu dienen, die Veränderung von Magnetfeldern zu registrieren und damit auf den Betriebszustand der Pumpe 110 Rückschlüsse erlauben. Außerdem weist die Vorrichtung 101 einen Sensor 130 zur Messung eines zweiten Betriebsparameters 520 der Vorrichtung 101 auf. In der in Figur 1 gezeigten Variante ist der Sensor 130 stromabwärts der Vorrichtung 101 an der Vorrichtung 101, in der unmittelbaren Nähe der Vorrichtung 101 oder nahe der Vorrichtung 101 an oder in der stromabwärts verlaufenden zweiten Leitung 155 angeordnet. Zudem weist die Vorrichtung ein Steuergerät 180 zur Steuerung der Zentrifugalpumpe 110 auf. Dieses Steuergerät 180 der Vorrichtung 101 ist mit der Pumpe 110, mit der Erfassungsvorrichtung 120 zur Erfassung des ersten Betriebsparameters 510 der Vorrichtung 101 und mit dem Sensor 130 zur Messung des zweiten Betriebsparameters 520 der Vorrichtung 101 verbunden. Das Steuergerät 180 steuert den Betrieb der Pumpe 110. Es ist denkbar, dass das Steuergerät 180 den Betrieb der gesamten Vorrichtung 101 steuert. Das Steuergerät 180 ist dazu eingerichtet, dass es im Betrieb der Vorrichtung 101 oder im Betrieb der Pumpe 110 Gasansammlungen aus dem hohlen Zentrum der Pumpe 110 entfernen kann. Darunter ist zu verstehen, dass das Innere der Pumpe 110 zunächst weitgehend mit der zu pumpenden Flüssigkeit gefüllt ist, sich jedoch im Flüssigkeitsförderbetrieb der Pumpe 110 in deren hohlem Zentrum eine Gasansammlung bildet, welche nun entfernt werden soll. Nach einer erfolgreichen Entfernung einer Gasansammlung befindet sich weniger Gas und mehr Flüssigkeit im Inneren der Pumpe 110. Das Steuergerät 180 ist dazu eingerichtet, dass es Gasansammlungen entfernen kann, indem in einem ersten Schritt zur Gasentfernung die Pumpe 110 betrieben wird und dabei der erste Betriebsparameter 510 der Pumpe 110 über die Zeit variiert wird. Der erste Betriebsparameter 510 kann beispielsweise rampenförmig oder stufenförmig variiert werden. Während die Pumpe 110 betrieben und der erste Betriebsparameter 510 variiert wird, wird auch der zweite Betriebsparameter 520 mit dem Sensor 130 gemessen. So erhält man Werte des zweiten Betriebsparameters 520 in Abhängigkeit von den Werten des ersten Betriebsparameters 510. Beispielsweise wird die Pumpendrehzahl pro Zeiteinheit als erster Betriebsparameter 510 variiert und dabei ein Körperschall als zweiter Betriebsparameter gemessen. In einem zweiten Schritt zur Entfernung von Gasansammlungen werden Extrema E in den zuvor aufgezeichneten Werten des zweiten Betriebsparameters 520 gesucht. Die Werte des ersten Betriebsparameters 510, die angelegen haben, als die extremalen Werte E des zweiten Betriebsparameters 520 gemessen wurden, werden in einem dritten Schritt ermittelt. In einem vierten Schritt zur Entfernung von Gasansammlungen - kurz: zur Gasentfernung - wird die Pumpe 110 so betrieben, dass sie für eine vorbestimmte Zeitdauer T erneut die Betriebsbedingungen annimmt, die vorlagen, als die extremalen Werte E des zweiten Betriebsparameters 520 gemessen wurden. Diese Bedingungen sind gekennzeichnet durch die im dritten Schritt ermittelten Werte des ersten Betriebsparameters 510. Es nimmt also die Pumpe 110 für eine vorbestimmte Zeitdauer, z.B. für 5 Sekunden, die Werte des ersten Betriebsparameters 510 an, die den extremalen Werten des zweiten Betriebsparameters 520 zugeordnet wurden.

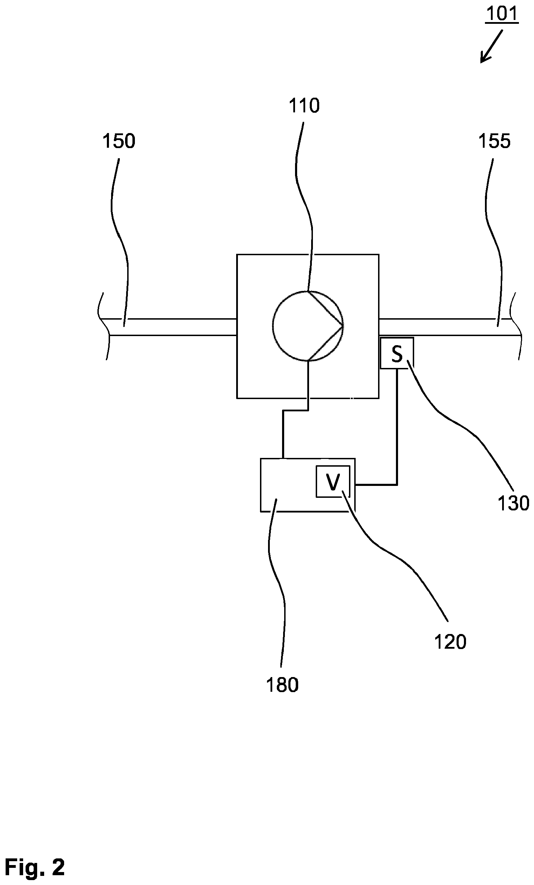

Figur 2 zeigt eine weitere Ausführung einer erfindungsgemäßen Pumpvorrichtung 101 schematisch. Figur 2 zeigt dieselben Bestandteile wie Figur 1, jedoch ist die Erfassungsvorrichtung 120 zur Erfassung des ersten Betriebsparameters 510 in unmittelbarer Nähe der Steuervorrichtung 180 oder als Bestandteil der Steuervorrichtung 180 angedeutet. Es ist beispielsweise denkbar, dass der erste Betriebsparameter ein Parameter der Pumpe 180 ist, den die Steuervorrichtung 180 im Betrieb ohnehin erfasst oder der der Steuervorrichtung 180 zumindest als Signal im Betrieb vorliegt. Das kann beispielsweise der Fall sein, wenn der erste Betriebsparameter ein Pumpenstrom oder ein Lagerstrom einer Pumpe 110, z.B. einer magnetisch levitierenden Impellerpumpe 110, ist. Es kann auch der Fall sein, wenn der Parameter eine Pumpenfrequenz oder die Umdrehungen einer Pumpe 110 pro Zeiteinheit sind, z.B. Umdrehungen pro Minute oder pro Sekunde.

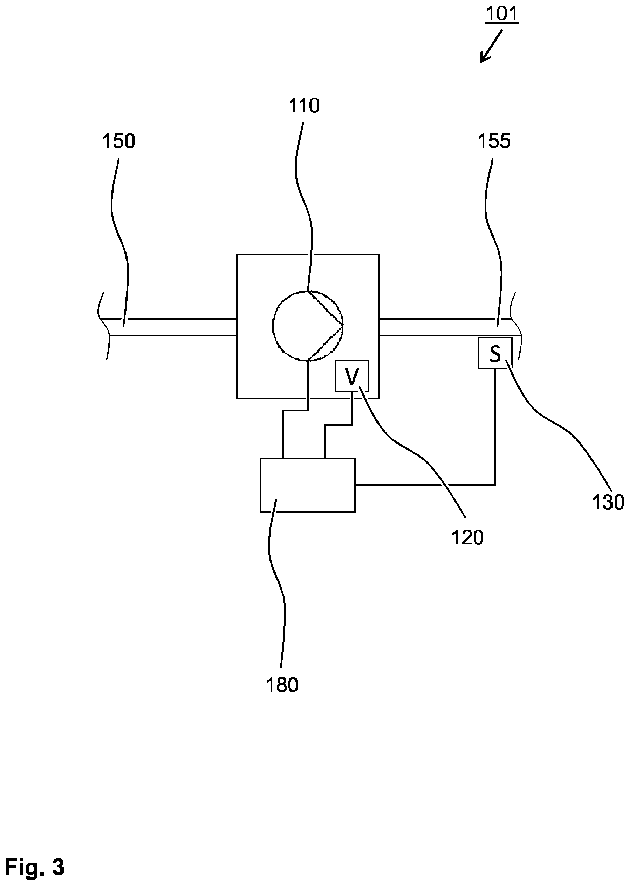

Figur 3 zeigt eine weitere Ausführung einer erfindungsgemäßen Pumpvorrichtung 101 schematisch. Figur 3 zeigt eine Pumpvorrichtung mit denselben Bestandteile wie die in Figur 1 gezeigte, jedoch ist der Sensor 130 zur Messung eines zweiten Betriebsparameters 520 im Unterschied zu der in Figur 1 gezeigten Ausführung hierbei beabstandet von der Pumpe 110 (und einem optionalen Pumpengehäuse, welches die Pumpe 110 umgibt) und beabstandet vom Steuergerät 180 an oder in einer von der Pumpe hinweg führenden Flüssigkeitsleitung angeordnet. Der Sensor befindet sich also stromabwärts der Pumpe in Bezug auf die Fließrichtung der gepumpten Flüssigkeit. Der hier gemessene zweite Betriebsparameter könnte beispielsweise ein Körperschall und / oder ein Schall in der Flüssigkeit sein.

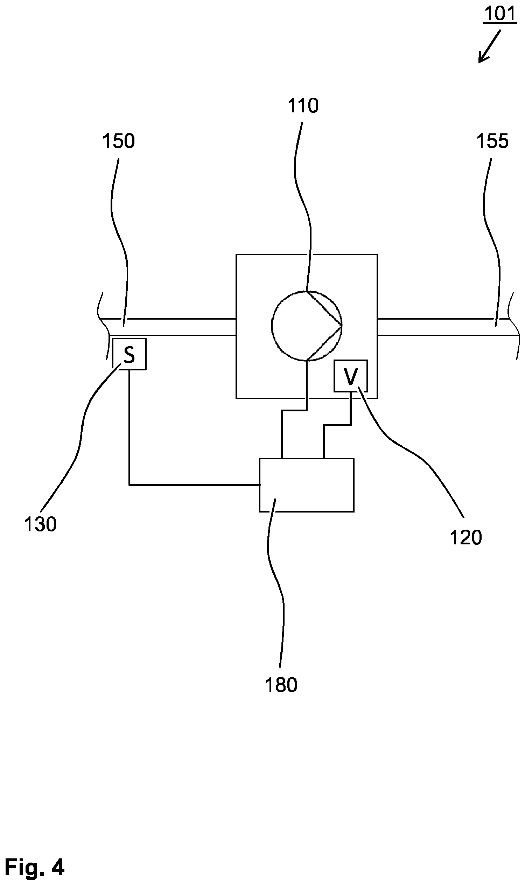

Figur 4 zeigt eine weitere Ausführung einer erfindungsgemäßen Pumpvorrichtung 101 schematisch. Figur 4 zeigt eine Pumpvorrichtung mit denselben Bestandteile wie die in Figur 1 gezeigte, jedoch ist der Sensor 130 zur Messung eines zweiten Betriebsparameters 520 im Unterschied zu der in Figur 1 gezeigten Ausführung hierbei beabstandet von der Pumpe 110 (und einem optionalen Pumpengehäuse, welches die Pumpe 110 umgibt) und beabstandet vom Steuergerät 180 an oder in einer zu der Pumpe hin führenden Flüssigkeitsleitung angeordnet. Der Sensor befindet sich also stromaufwärts der Pumpe in Bezug auf die Fließrichtung der gepumpten Flüssigkeit. Der hier gemessene zweite Betriebsparameter könnte beispielsweise ein Körperschall und / oder ein Schall in der Flüssigkeit sein.

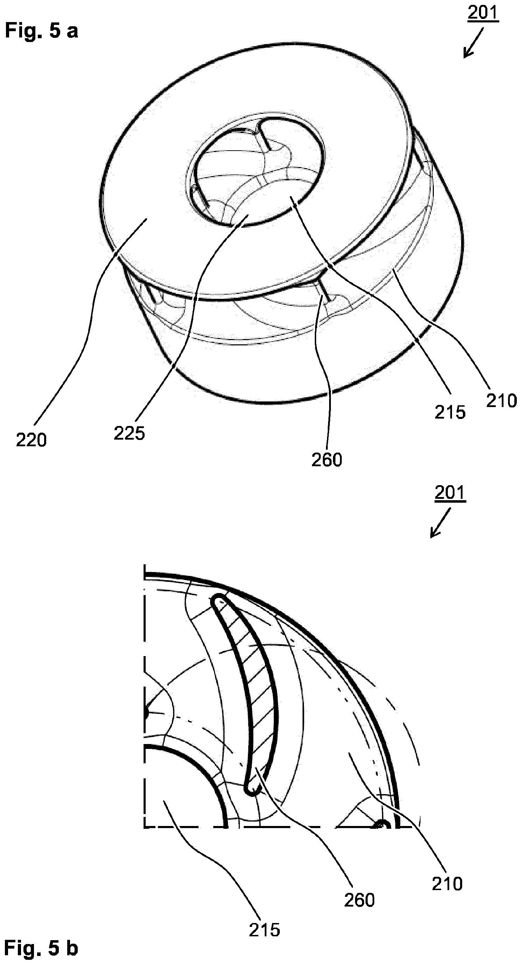

Figur 5 zeigt ein beispielhaftes Pumpenrad 201 mit hohlem Zentrum zum radialen Pumpen. Eine Pumpvorrichtung 101 zum Pumpen von Flüssigkeiten mit Zentrifugalpumpe 110 mit radial pumpendem Pumpenrad 201 mit hohlem Zentrum könnte beispielsweise ein derartiges Pumpenrad 201 aufweisen. Dabei zeigt Figur 5 a eine isometrische Ansicht eines Pumpenrads 201 und Figur 5 b eine Teilansicht einer Schnittzeichnung desselben Pumpenrads 201. Im Wesentlichen kann man das beispielhafte Pumpenrad 201 beschreiben als zwei im wesentlichen kreisförmige Scheiben 210, 220, die parallel zueinander liegen. In einer Variante können die kreisförmigen Scheiben 210, 220 jeweils ein zentrales Loch 215, 225, d.h. ein Loch 215, 225 am und um den Mittelpunkt aufweisen. In einer alternativen Variante ist zumindest eine der kreisförmigen Scheiben 210, 220 als massive Kreisscheibe ausgeführt und die andere kreisförmige Scheibe 220, 210 weist ein zentrales Loch 225, 215 auf. Die Darstellung versucht, beide Varianten zugleich anzudeuten. In einer weiteren, hier nicht gezeigten alternativen Variante weist das Pumpenrad nur eine Scheibe 210, 220 auf und die Schaufelblätter 260 sind jeweils am scheibenfernen Ende frei. In einer weiteren, hier nicht gezeigten alternativen Variante weist das Pumpenrad zwei Scheiben 210, 220 auf, die keine zentrale Ausnehmung aufweisen. Die beiden Scheiben 210, 220 sind verbunden mit Pumpenschaufeln 260, die sich als radiale Streben zwischen der ersten Scheibe 210 und der zweiten Scheibe 220 erstrecken können. Die gezeigten Schaufeln 260 weisen einen auf die Rotationsache bezogen radialen Anteil, einen tangentialen Anteil und eine Biegung auf, sind also nicht gerade und verlaufen also nicht rein radial und nicht rein tangential, sondern "diagonal" - sozusagen mit radialem und tangentialem Anteil. Das hohle Zentrum der Pumpe 110 erstreckt sich insbesondere in dem Bereich, der in der gezeigten Darstellung von dem Loch 215 in der ersten Scheibe 210 und dem Loch 225 in der zweiten Scheibe 220 aufgespannt wird. Kennzeichnend für das hohle Zentrum der Pumpe 110 ist jedoch in allen Ausführungsformen, dass die Pumpenschaufeln 260 (auch Schaufelblätter) gerade nicht bis in das hohle Zentrum reichen. Das hohle Zentrum der Pumpe 110 ist also der Bereich an der Rotationsachse des Pumpenrads und / oder unmittelbar darum, der frei ist von Pumpenschaufeln. Dieser Bereich ist auch nicht gefüllt von einem massiven Körper, sondern er kann frei von der zu pumpenden Flüssigkeit und eben auch von unerwünschten Gasansammlungen darin durchströmt werden. Es ist denkbar, dass direkt auf der Rotationsachse ein massiver Körper, z.B. eine Achse oder Welle verläuft. In dem Fall ist das hohle Zentrum der Bereich zwischen der Achse oder Welle und den Pumpenschaufeln, der frei von der zu pumpenden Flüssigkeit durchströmt werden kann.

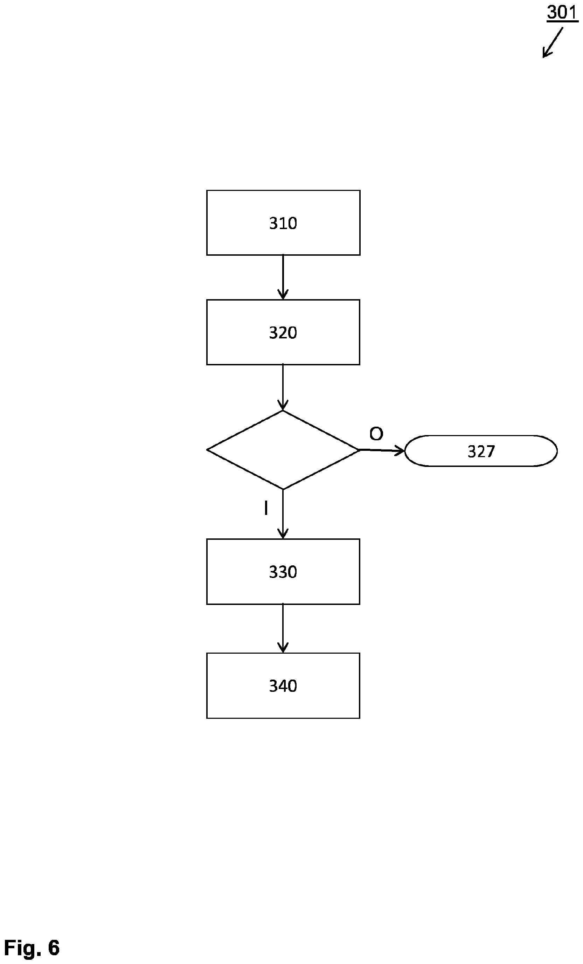

Figur 6 zeigt ein Ablaufdiagramm eines erfindungsgemäßen Verfahrens 301 zur Entfernung von Gasansammlungen aus dem Inneren von Zentrifugalpumpen 110 mit radial pumpendem Pumpenrad 201mit hohlem Zentrum. Es wird ein Ablauf gezeigt, der optional nach der Bestimmung von Extrema abbrechen kann, wenn keine Extrema ermittelt wurden. Diese optionale Überprüfung muss nicht in allen Varianten des Verfahrens vorkommen. Das Verfahren weist die folgenden Verfahrensschritte auf:
- Betreiben 310 einer Zentrifugalpumpe, wobei ein erster Betriebsparameter 510 der Pumpe 110 dahingehend variiert wird, dass der erste Betriebsparameter 510 nacheinander eine Reihe von verschiedenen vorbestimmten Werten einnimmt, Währenddessen wird zu jedem Wert des ersten Betriebsparameters 510 zumindest ein Wert eines zweiten Betriebsparameters 520 aufgezeichnet.
- Bestimmung 320 von lokalen und / oder globalen Extrema E der zuvor aufgezeichneten Werte des zweiten Betriebsparameters 520, Optional: Überprüfung, ob Extrema E bestimmt werden konnten. Ergibt die Überprüfung, dass zumindest ein Extremum E vorliegt I, wird das Verfahren mit der Bestimmung 330 der zugehörigen Werde des ersten Betriebsparameters 510 fortgesetzt. Ergibt die Überprüfung, dass keine Extrema E vorliegen O, wird das Verfahren abgebrochen 327.
- Bestimmung 330 derjenigen Werte des ersten Betriebsparameters 510, welche den zuvor ermittelt Extrema E des zweiten Betriebsparameter 520 zugeordnet sind,
- Betreiben 340 der Pumpe so, dass sie für eine vorbestimmte Zeitdauer einen der den Extrema E des zweiten Betriebsparameters 520 zugeordneten Werte des ersten Betriebsparameters 510 annimmt.

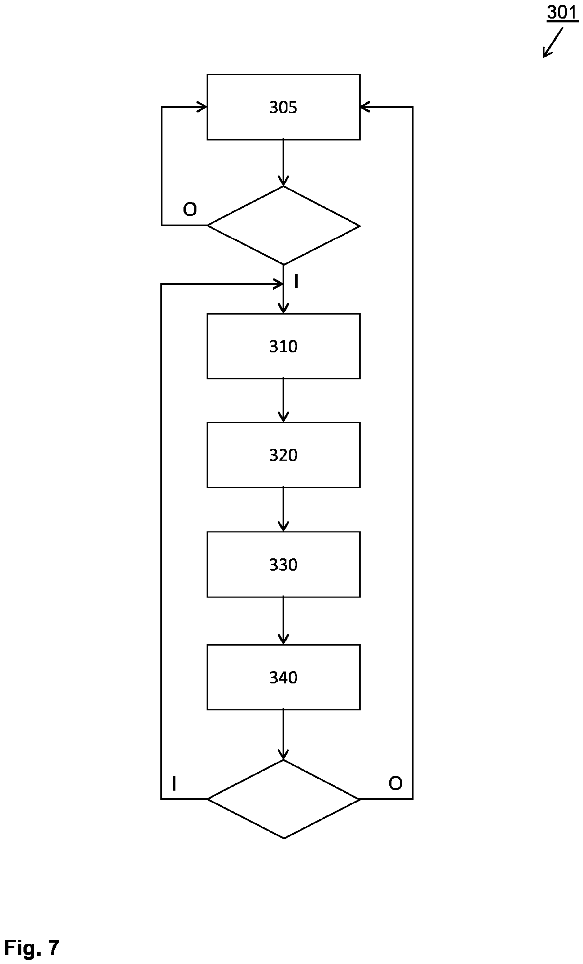

Figur 7 zeigt eine weitere beispielhafte Ausführungsform eines erfindungsgemäßen Verfahrens 301 zur Entfernung von Gasansammlungen aus dem Inneren von Zentrifugalpumpen 110 mit radial pumpendem Pumpenrad 201mit hohlem Zentrum als Ablaufdiagramm. Im Unterschied zu der in Figur 6 gezeigten Variante wird hier die Pumpe 110 dauerhaft so betrieben, dass ein Betriebsparameter der Vorrichtung 101 im regulären Betrieb kontinuierlich oder in vorbestimmten zeitlichen Abständen überprüft 305 wird. Der überprüfte Parameter kann beispielsweise der erste Betriebsparameter 510 sein. Anhand der Werte oder der Veränderung der Werte dieses Betriebsparameters wird erkannt, wenn sich Gasansammlungen im Inneren der Pumpe 110 befinden. Dazu können die Werte des Betriebsparameters beispielsweise mit vorbestimmten Erkennungsprofilen verglichen werden. Oder das Überschreiten oder Unterschreiten eines vorbestimmten Schwellenwerts des Parameters wird als Anzeichen, dass eine Gasansammlung im Inneren der Pumpe 110 vorliegt, herangezogen. Alternativ oder zusätzlich kann ein Verlauf der Entwicklung des Parameters, also dessen Trend, zur Ermittlung, ob eine Gasansammlung im Inneren der Pumpe 110 vorliegt, herangezogen werden. Wird bei der Überprüfung 305 des Parameters keine Gasansammlung erkannt O, so wird der reguläre Betrieb fortgesetzt, was eine Fortführung der Überprüfung 305 einschließt. Wird bei der Überprüfung 305 des Parameters eine Gasansammlung erkannt I, so werden Schritte zur Entfernung der Gasansammlung eingeleitet, wie in Zusammenhang mit Figur 6 beschrieben: Variieren des ersten Parameters 310, Erfassen 320 von erstem Parameter 510 und zweitem Parameter 520 dabei, Bestimmen 330 von Extrema E es zweiten Parameters 520, Bestimmen von den den Extrema E des zweiten Parameters 520 zugeordneten Werten 510(E) des ersten Parameters 510, Betreiben der Pumpe 110 für eine vorbestimmte Zeitdauer T, wobei die Pumpe 110 einen ermittelten, den Extrema E des zweiten Parameters 520 zugeordneten Werte 510(E) des ersten Parameters 510 annimmt. In der hier gezeigten Variante wird nach Durchlaufen dieser Schritte optional zusätzlich überprüft, ob noch immer eine Gasansammlung in der Pumpe 110 erkannt wird. Wenn weiterhin eine Gasansammlung im Inneren der Pumpe 110 festgestellt I wird, werden die vier Schritte 310, 320, 330, 340 erneut durchlaufen, wobei die Extrema E nicht dieselben Werte wie im vorigen Durchlauf einnehmen müssen. Eine Veränderung der Extrema E kann als Teilerfolg der Gasentfernung gewertet werden. Wird keine Gasansammlung mehr erkannt O, so wird der reguläre Betrieb fortgesetzt.

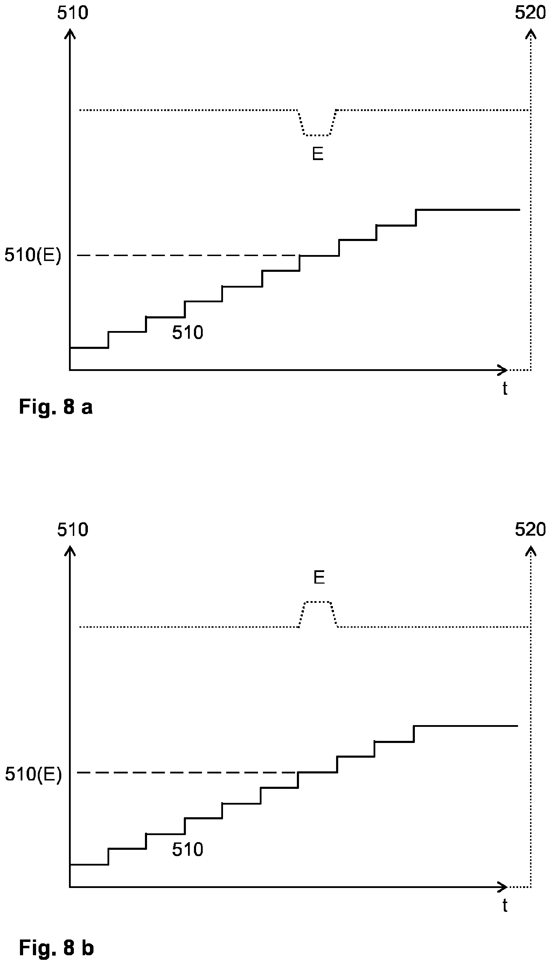

Figur 8 zeigt beispielhaft das Variieren des ersten Betriebsparameters 510 über die Zeit t in Form einer idealisierten Stufenfunktion (als durchgezogene Linie), bei der der erste Betriebsparameter 510 über die Zeit t hinweg ansteigende Werte annimmt. Dabei zeigt Figur 8 a beispielhaft ein Extremum E eines beispielhaften simultan aufgenommenen zweiten Betriebsparameters 520, welches ein Minimum ist, und Figur 8 b zeigt einen beispielhaften simultanen Verlauf eines beispielhaften zweiten Betriebsparameters 520, welches ein Extremum E in Form eines Maximums ausbildet. In beiden Graphen entspricht die vertikale Achse links der Größe des Werts des ersten Betriebsparameters 510 in willkürlichen Einheiten und die horizontale Achse entspricht der Zeit t. Die Kurve in durchbrochener Linie zeigt jeweils die Veränderung des zweiten Betriebsparameters 520 während der erste Betriebsparameter 510 wie gezeigt variiert wird. Die rechte vertikale Achse in durchbrochener Linie zeigt die Größe des Werts des zweiten Betriebsparameters 520. In beiden Graphen tritt ein Extremum E des zweiten Betriebsparameters 520 auf, wenn der erste Betriebsparameter 510 einen bestimmten Wert 510(E) annimmt.

## Patentansprüche

1. Pumpvorrichtung (101) zum Pumpen von Flüssigkeiten aufweisend
a. eine Zentrifugalpumpe (110) mit radial pumpendem Pumpenrad (201) mit hohlem Zentrum, wobei der Pumpe (110) im Betrieb zu jedem Zeitpunkt ein Wert eines ersten Betriebsparameters (510) zugeordnet werden kann,
b. eine Erfassungsvorrichtung (120) zur Erfassung des ersten Betriebsparameters (510),
c. einen Sensor (130) zur Messung eines zweiten Betriebsparameters (520),
d. und ein Steuergerät (180) zur Steuerung der Zentrifugalpumpe(110), das mit der Pumpe (110), der Erfassungsvorrichtung (120) zur Erfassung des ersten Betriebsparameters (510) und mit dem Sensor (130) verbunden ist, ferner dazu eingerichtet, dass es den Betrieb der Pumpe (110) steuert und dass es im Flüssigkeitsförderbetrieb der Pumpe (110) Gasansammlungen aus dem hohlen Zentrum der Pumpe (110) entfernen kann, indem
∘ in einem ersten Schritt (310) zur Gasentfernung die Pumpe (110) so betrieben wird, dass zumindest der erste Betriebsparameter (510) der Pumpe (110) derart über die Zeit (t) variiert wird, dass der erste Betriebsparameter (510) nacheinander eine Reihe von verschiedenen vorbestimmten Werten einnimmt,
▪ und dass währenddessen zu den Werten des ersten Betriebsparameters (510) zugeordnete Werte des zweiten Betriebsparameters (520) aufgezeichnet werden,
∘ in einem zweiten Schritt (320) zur Gasentfernung lokale und / oder globale Extrema (E) der zuvor aufgezeichneten Werte des zweiten Betriebsparameters (520) bestimmt werden,
∘ in einem dritten Schritt (330) zur Gasentfernung diejenigen Werte (510(E)) des ersten Betriebsparameters (510) bestimmt werden, welche den zuvor ermittelt Extrema (E) des zweiten Betriebsparameters (520) zugeordnet sind,
∘ in einem vierten Schritt (340) zur Gasentfernung die Pumpe (110) so betrieben wird, dass sie für eine vorbestimmte Zeitdauer (T) einen der den Extrema (E) des zweiten Betriebsparameters (520) zugeordneten Werte des ersten Betriebsparameters (510) annimmt.

2. Pumpvorrichtung (101) nach Anspruch 1, so eingerichtet, dass der erste Betriebsparameter (510) eine Pumpendrehzahl pro Zeiteinheit oder eine Pumpenfrequenz der Zentrifugalpumpe (110) ist.

3. Pumpvorrichtung (101) nach einem der vorangehenden Ansprüche, wobei der zweite Betriebsparameter (520) direkt oder indirekt an der Pumpvorrichtung gemessener Körperschall oder Schall in der zu pumpenden Flüssigkeit ist.

4. Pumpvorrichtung (101) nach einem der vorangehenden Ansprüche, wobei die Pumpe elektrisch angetrieben ist und ein erster Betriebsparameter (510) ein elektrischer Betriebsstrom der Pumpe (110) oder ein Betriebsstrom pro Drehzahl der Pumpe (110) ist.

5. Pumpvorrichtung (101) nach Anspruch 4, wobei die Pumpe (110) eine magnetisch levitierend gelagerte Impellerpumpe (110) ist und ein erster Betriebsparameter (510) ein Lagerstrom der Pumpe (110) oder ein Lagerstrom pro Drehzahl der Pumpe (110) ist.

6. Pumpvorrichtung (101) nach einem der vorangehenden Ansprüche, wobei das Steuergerät (180) dazu eingerichtet ist, den vierten Schritt (340) für mehrere unterschiedliche den Extrema (E) des zweiten Betriebsparameters (520) zugeordneten Werte des ersten Betriebsparameters (510) durchzuführen.

7. Pumpvorrichtung (101) nach einem der vorangehenden Ansprüche, wobei das Steuergerät (180) dazu eingerichtet ist, nach der Durchführung des vierten Schrittes (340) erneut unerwünschte Gasansammlungen zu entfernen, indem die Schritte zur Entfernung von Gasansammlung vom ersten Schritt (310) bis zum vierten Schritt (340) erneut durchlaufen werden, wobei die ausgezeichneten Werte des ersten Betriebsparameters (510) verschiedener Durchläufe unterschiedlich sein können.

8. Pumpvorrichtung (101) nach einem der vorangehenden Ansprüche, wobei das Steuergerät (180) dazu eingerichtet ist, den ersten Betriebsparameter (510) im ersten Schritt (310) linear oder in Sprüngen mit festen Abständen oder als Intervallschachtelung, welche sich einem vorbestimmten Wert in vorbestimmter Geschwindigkeit näher, zu variieren.

9. Pumpvorrichtung (101) nach einem der vorangehenden Ansprüche, wobei das Steuergerät (180) dazu eingerichtet ist, die Gegenwart einer unerwünschten Gasansammlung im Inneren der Pumpe (110) zu erkennen, indem beim Betrieb der Pumpe (110) kontinuierlich oder in vorbestimmten Zeitintervallen der erste Betriebsparameter (510) der Pumpe (110) überwacht (305) und dessen Veränderung über die Zeit mit vorbestimmten Erkennungsprofilen verglichen wird, und bei Erkennen einer Gasansammlung das Entfernen von Gasansammlungen durchzuführen.

10. Pumpvorrichtung (101) nach einem der vorangehenden Ansprüche, wobei das Steuergerät (180) dazu eingerichtet ist, den normalen Flüssigkeitsförderbetrieb fortzusetzen, wenn beim Entfernen von Gasansammlungen im zweiten Schritt keine Extrema (E) aufgefunden worden sind.

11. Pumpvorrichtung (101) zur Beförderung von Blut und / oder medizinischer Behandlungsflüssigkeit und / oder medizinischer Behandlungs-Abfallflüssigkeit nach einem der Ansprüche 1 bis 10.

12. Verfahren (301) zur Entfernung von Gasansammlungen aus dem Inneren von Zentrifugalpumpen (110) mit radial pumpendem Pumpenrad (201)mit hohlem Zentrum mit zumindest den folgenden Verfahrensschritten:
a. Betreiben (310) einer Zentrifugalpumpe, wobei ein erster Betriebsparameter (510) der Pumpe (110) dahingehend variiert wird, dass der erste Betriebsparameter (510) nacheinander eine Reihe von verschiedenen vorbestimmten Werten einnimmt,
i. währenddessen zu jedem Wert des ersten Betriebsparameters (510) zumindest ein Wert eines zweiten Betriebsparameters (520) aufgezeichnet wird,
b. Bestimmung (320) von lokalen und / oder globalen Extrema (E) der zuvor aufgezeichneten Werte des zweiten Betriebsparameters (520),
c. Bestimmung (330) derjenigen Werte des ersten Betriebsparameters (510), welche den zuvor ermittelt Extrema (E) des zweiten Betriebsparameter (520) zugeordnet sind,
d. Betreiben (340) der Pumpe so, dass sie für eine vorbestimmte Zeitdauer einen der den Extrema (E) des zweiten Betriebsparameters (520) zugeordneten Werte des ersten Betriebsparameters (510) annimmt.

## Claims

1. Pump device (101) for pumping liquids, comprising
a. a centrifugal pump (110) having a radially pumping pump wheel (201) with a hollow centre, wherein the pump (110), during operation, can at any time be assigned a value of a first operating parameter (510),
b. a detecting device (120) for detecting the first operating parameter (510),
c. a sensor (130) for measuring a second operating parameter (520),
d. and a control unit (180) for controlling the centrifugal pump (110), said control unit (180) being connected to the pump (110), to the detecting device (120) for detecting the first operating parameter (510) and to the sensor (130) and moreover being configured such that it controls the operation of the pump (110) and can remove accumulations of gas from the hollow centre of the pump (110) during the liquid-conveying operation of the pump (110), by
o operating the pump (110), in a first step (310) for removal of gas, such that at least the first operating parameter (510) of the pump (110) is varied over time (t) in such a way that the first operating parameter (510) assumes a series of different predetermined values one after another,
• and that meanwhile values of the second operating parameter (520) that are assigned to the values of the first operating parameter (510) are recorded,
o determining, in a second step (320) for removal of gas, local and/or global extreme values (E) of the previously recorded values of the second operating parameter (520),
o determining, in a third step (330) for removal of gas, those values (510(E)) of the first operating parameter (510) that are assigned to the previously determined extreme values (E) of the second operating parameter (520),
o operating the pump (110), in a fourth step (340) for removal of gas, such that it assumes, for a predetermined period of time (T), one of the values of the first operating parameter (510) that are assigned to the extreme values (E) of the second operating parameter (520).

2. Pump device (101) according to Claim 1, configured such that the first operating parameter (510) is a pump rotational speed per unit of time or a pump frequency of the centrifugal pump (110).

3. Pump device (101) according to one of the preceding claims, wherein the second operating parameter (520) is structure-borne sound, measured directly or indirectly on the pump device, or sound in the liquid to be pumped.

4. Pump device (101) according to one of the preceding claims, wherein the pump is driven electrically, and a first operating parameter (510) is an electrical operating current of the pump (110) or an operating current per rotational speed of the pump (110).

5. Pump device (101) according to Claim 4, wherein the pump (110) is an impeller pump (110) mounted to levitate magnetically, and a first operating parameter (510) is a bearing current of the pump (110) or a bearing current per rotational speed of the pump (110).

6. Pump device (101) according to one of the preceding claims, wherein the control unit (180) is configured to carry out the fourth step (340) for a plurality of different values of the first operating parameter (510) that are assigned to the extreme values (E) of the second operating parameter (520).

7. Pump device (101) according to one of the preceding claims, wherein the control unit (180) is configured to again remove unwanted accumulations of gas after carrying out the fourth step (340) and to do so by again running through the steps for removal of accumulation of gas from the first step (310) to the fourth step (340), wherein the recorded values of the first operating parameter (510) of different runs may be different.

8. Pump device (101) according to one of the preceding claims, wherein the control unit (180) is configured to vary the first operating parameter (510) in the first step (310) in a linear fashion or in jumps at fixed intervals or as interval nesting, approaching a predetermined value at predetermined speed.

9. Pump device (101) according to one of the preceding claims, wherein the control unit (180) is configured to detect the presence of an unwanted accumulation of gas in the interior of the pump (110) by monitoring (305) the first operating parameter (510) of the pump (110) continuously or at predetermined intervals during operation of the pump (110) and by comparing any change in said operating parameter over time with predetermined detection profiles, and by carrying out the removal of accumulations of gas if an accumulation of gas is detected.

10. Pump device (101) according to one of the preceding claims, wherein the control unit (180) is configured to continue the normal liquid-conveying operation if no extreme values (E) have been discovered during the removal of accumulations of gas in the second step.

11. Pump device (101) for transporting blood and/or medical treatment liquid and/or medical treatment waste liquid according to one of Claims 1 to 10.

12. Method (301) for removing accumulations of gas from the interior of centrifugal pumps (110) having a radially pumping pump wheel (201) with a hollow centre, with at least the following method steps:
a. operating (310) a centrifugal pump, wherein a first operating parameter (510) of the pump (110) is varied such that the first operating parameter (510) assumes a series of different predetermined values one after another,
i. meanwhile at least one value of a second operating parameter (520) is recorded for each value of the first operating parameter (510),
b. determining (320) local and/or global extreme values (E) of the previously recorded values of the second operating parameter (520),
c. determining (330) those values of the first operating parameter (510) that are assigned to the previously determined extreme values (E) of the second operating parameter (520),
d. operating (340) the pump such that it assumes, for a predetermined period of time, one of the values of the first operating parameter (510) that are assigned to the extreme values (E) of the second operating parameter (520).

## Revendications

1. Dispositif de pompage (101) servant au pompage de liquides comprenant
a. une pompe centrifuge (110) dotée d'une roue de pompe (201) pompant radialement présentant un centre creux, une valeur d'un premier paramètre de fonctionnement (510) pouvant être associée à la pompe (110) à chaque instant lors du fonctionnement,
b. un dispositif de détection (120) servant à la détection du premier paramètre de fonctionnement (510),
c. un capteur (130) servant à la mesure d'un deuxième paramètre de fonctionnement (520),
d. et un appareil de commande (180) servant à la commande de la pompe centrifuge (110), lequel est connecté à la pompe (110), au dispositif de détection (120) servant la détection du premier paramètre de fonctionnement (510) et au capteur (130), en outre conçu de telle sorte qu'il commande le fonctionnement de la pompe (110) et de telle sorte qu'il peut éliminer des accumulations de gaz du centre creux de la pompe (110) pendant le fonctionnement de refoulement de liquide de la pompe (110), par le fait que
o dans une première étape (310) d'élimination de gaz, la pompe (110) fonctionne de telle sorte qu'au moins le premier paramètre de fonctionnement (510) de la pompe (110) est modifié au cours du temps (t) de telle sorte que le premier paramètre de fonctionnement (510) adopte tour à tour une série de différentes valeurs prédéfinies,
▪ et de telle sorte que pendant ce temps des valeurs du deuxième paramètre de fonctionnement (520) associées aux valeurs du premier paramètre de fonctionnement (510) sont enregistrées,
o dans une deuxième étape (320) d'élimination de gaz, des extrema locaux et/ou globaux (E) des valeurs préalablement enregistrées du deuxième paramètre de fonctionnement (520) sont déterminées,
o dans une troisième étape (330) d'élimination de gaz, les valeurs (510(E)) du premier paramètre de fonctionnement (510) qui sont associées aux extrema (E) préalablement déterminés du deuxième paramètre de fonctionnement (520) sont déterminées,
o dans une quatrième étape (340) d'élimination de gaz, la pompe (110) fonctionne de telle sorte qu'elle adopte pendant une durée prédéfinie (T) l'une des valeurs du premier paramètre de fonctionnement (510) associées aux extrema (E) du deuxième paramètre de fonctionnement (520).

2. Dispositif de pompage (101) selon la revendication 1, conçu de telle sorte que le premier paramètre de fonctionnement (510) est une vitesse de rotation de pompe par unité de temps ou une fréquence de pompe de la pompe centrifuge (110).

3. Dispositif de pompage (101) selon l'une des revendications précédentes, le deuxième paramètre de fonctionnement (520) étant le bruit de structure ou le bruit, mesuré directement ou indirectement au niveau du dispositif de pompage, dans le liquide à pomper.

4. Dispositif de pompage (101) selon l'une des revendications précédentes, la pompe étant entraînée électriquement et un premier paramètre de fonctionnement (510) étant un courant de fonctionnement électrique de la pompe (110) ou un courant de fonctionnement par vitesse de rotation de la pompe (110).

5. Dispositif de pompage (101) selon la revendication 4, la pompe (110) étant une pompe à rotor (110) lévitant magnétiquement et un premier paramètre de fonctionnement (510) étant un courant de palier de la pompe (110) ou un courant de palier par vitesse de rotation de la pompe (110) .

6. Dispositif de pompage (101) selon l'une des revendications précédentes, l'appareil de commande (180) étant conçu pour mettre en œuvre la quatrième étape (340) pour plusieurs valeurs différentes du premier paramètre de fonctionnement (510), associées aux extrema (E) du deuxième paramètre de fonctionnement (520).

7. Dispositif de pompage (101) selon l'une des revendications précédentes, l'appareil de commande (180) étant conçu pour, après la mise en œuvre de la quatrième étape (340), éliminer à nouveau des accumulations de gaz indésirables, par le fait que les étapes d'élimination d'accumulation de gaz de la première étape (310) à la quatrième étape (340) sont réalisées à nouveau, les valeurs enregistrées du premier paramètre de fonctionnement (510) de différentes itérations pouvant être différentes.

8. Dispositif de pompage (101) selon l'une des revendications précédentes, l'appareil de commande (180) étant conçu pour modifier le premier paramètre de fonctionnement (510) dans la première étape (310) de manière linéaire ou par bonds à des intervalles fixes ou comme emboîtement d'intervalles, lesquels se rapprochent d'une valeur prédéfinie à une vitesse prédéfinie.

9. Dispositif de pompage (101) selon l'une des revendications précédentes, l'appareil de commande (180) étant conçu pour détecter la présence d'une accumulation de gaz indésirable à l'intérieur de la pompe (110), par le fait que lors du fonctionnement de la pompe (110), le premier paramètre de fonctionnement (510) de la pompe (110) est surveillé (305) de manière continue ou à des intervalles de temps prédéfinis et sa variation au cours du temps est comparée à des profils de détection prédéfinis, et pour mettre en œuvre l'élimination d'accumulations de gaz lors de la détection d'une accumulation de gaz.

10. Dispositif de pompage (101) selon l'une des revendications précédentes, l'appareil de commande (180) étant conçu pour continuer le fonctionnement de refoulement de liquide normal si aucuns extrema (E) n'ont été découverts lors de l'élimination d'accumulations de gaz dans la deuxième étape.

11. Dispositif de pompage (101) pour le transport de sang et/ou de liquide de traitement médical et/ou de déchets liquides de traitement médicaux selon l'une des revendications 1 à 10.

12. Procédé (301) d'élimination d'accumulations de gaz de l'intérieur de pompes centrifuges (110) dotées d'une roue de pompe (201) pompant radialement présentant un centre creux comportant au moins les étapes de procédé suivantes :
a. fonctionnement (310) d'une pompe centrifuge, un premier paramètre de fonctionnement (510) de la pompe (110) étant modifié de telle sorte que le premier paramètre de fonctionnement (510) adopte tour à tour une série de différentes valeurs prédéfinies,
i. pendant ce temps, pour chaque valeur du premier paramètre de fonctionnement (510), au moins une valeur d'un deuxième paramètre de fonctionnement (520) est enregistrée,
b. détermination (320) d'extrema locaux et/ou globaux (E) des valeurs préalablement enregistrées du deuxième paramètre de fonctionnement (520),
c. détermination (330) des valeurs du premier paramètre de fonctionnement (510) qui sont associées aux extrema (E) préalablement déterminés du deuxième paramètre de fonctionnement (520),
d. fonctionnement (340) de la pompe de telle sorte qu'elle adopte pendant une durée prédéfinie l'une des valeurs du premier paramètre de fonctionnement (510) associées aux extrema (E) du deuxième paramètre de fonctionnement (520).
